# EUROPEAN PATENT APPLICATION

(11) **EP 3 763 330 A1**
(43) Date of publication of application: **13.01.2021**
(21) Application number: 19185491.8
(22) Date of filing: 10.07.2019
(51) Int. Cl.: A61F 2/24, A61F 2/07, A61F 2/90

(54) **IMPLANT, DELIVERY DEVICE AND METHOD OF DELIVERING AN IMPLANT**

(71) Applicant: P+F Cardiovascular GmbH, 1190 Wien (AT)
(72) Inventor: Einhellig, Siegfried, 1190 Wien (AT); Agreli, Guillherme, 1190 Wien (AT); Kiss, Katharina, 1190 Wien (AT)
(74) Representative: Manitz Finsterwald Patent- und Rechtsanwaltspartnerschaft mbB

(57) **Abstract**

The present invention relates to an implant for placement in a cardiac vessel, the implant being able to be expanded from an un-deployed state to an expanded state in which the implant has a larger outer diameter than in the storage state, the implant having a main body with first and second ends and a central portion arranged between the first and second ends, the central portion of the implant having a larger outer diameter than the first and second ends of the implant when the implant is in the expanded state. The invention further relates to a delivery device for an implant and to a method of delivering an implant.

## Description

The present invention relates to an implant for placement in a cardiac vessel, especially an implant for aortic root reconstruction and aortic valve replacement, the implant being able to be expanded from an un-deployed state to an expanded state in which the implant has a larger outer diameter than in the storage state, the implant having a main body with first and second ends and a central portion arranged between the first and second ends, the central portion of the implant having a larger outer diameter than the first and second ends of the implant when the implant is in the expanded state. The invention further relates to a delivery device for an implant and to a method of delivering an implant.

Patients suffering from acute type A dissection, ascending aorta aneurysm, or other conditions that require urgent intervention, such as, ascending aorta replacement are frequently subjected to the so-called Bentall-de Bono procedure during which a part of the ascending aorta, the aortic valve, and the aortic root is removed while a patient is placed on bypass.

Following this a replacement valve is fitted and a graft is sewn in place at the aortic valve together with the replacement valve. Thereafter a portion of the ascending aorta is attached to the graft and the coronary arteries are likewise sewn into the graft.
Such surgical procedures generally take several hours to perform and the re-implantation of the coronary arteries is prone to complications.
Moreover, the time to treatment is a crucial factor in reducing the mortality rate of acute aortic syndromes, i.e. a reduction in the time to treatment can lead to a reduced mortality rate. It has also been found that the replacement of the aortic root should be avoided as far as possible, since this can lead to further complications.

For this reason it is an object of the present invention to make available a device in ascending aorta reconstruction with aortic valve replacement, as an endovascular/ percutaneous/ minimally invasive alternative to the Bentall-de Bono procedure by means of which the complications which may arise during the invasive Bentall-de Bono procedure can be minimized. It is a further object of the present invention to reduce the time to treatment of acute aortic syndromes.

This object is satisfied by an implant having the features of claim 1.

Such an implant for placement in a cardiac vessel is able to be expand from an un-deployed state to an expanded state in which the implant has a larger outer diameter than in the storage state, with the implant having a main body with first and second ends and a central portion arranged between the first and second ends, the central portion of the implant having a larger outer diameter than the first and second ends of the implant when the implant is in the expanded state, the implant further comprising:
- a replacement valve for placement at the aortic valve arranged at the first end of the implant,
- a self-expandable aortic stent frame for placement at the ascending aorta arranged at a second end of the implant; and
- two or more internal branches for connection to further vessels, the internal branches extending from the implant.

The implant is thus a combination of a transcatheter aortic valve, an ascending aorta stent graft, and branches configured to be connected to covered coronary stents. Thereby the number of complications which may arise during the attachment of the coronary arteries and the aortic root to the stent graft on performing the Bentall-de Bono procedure can be reduced.

Moreover, a patient does not have to be placed on bypass during the procedure which further reduces the possibility of bleeding, arrhythmias, infections of the chest wound, memory loss, kidney problems, stoke or even a heart attack.

The implant is designed to be implanted using the arterial femoral access which can be conducted minimally invasive. In this way the recovery time of a patient can be significantly reduced leading to reduced cost of hospitalization.

The implant may be reliably used to treat patients suffering from acute type A dissection, ascending aorta aneurysm, or other conditions that require urgent intervention.

By way of such a device the time to treatment can be significantly reduced leading to reduced mortality rates for acute aortic syndromes, provided the patient is within the vicinity of a medical facility. This is because the valve replacement device in the form of the implant can save time to treatment when this is transported to the heart via the arterial femoral access. This is particularly the case if the valve is preloaded at a delivery device and the equipment is ready to use from a shelf.

The main body of the implant may be completely covered with a polymeric or biological material, optionally wherein the internal branches and in particular the complete implant is covered with material. In this way damage to the tissue at which the implant is placed can be avoided, reducing the number of complications that could be present without such a layer of material.

Respective first exits of the internal branches may have an opening that may into the central portion of the implant and respective second exits of the internal branches may have an opening that may open into an area surrounding the implant. In this way a blood flow stemming from the coronary arteries can be introduced into the implant without having to cut the arteries from the aortic root on treating acute type A dissection, ascending aorta aneurysm, or other conditions that require urgent intervention.

The valve may be a biological dry tissue valve. Such a valve allows the preloading of the implant within a delivery system. Such procedures save time during implantation and increase safety, avoid making mistakes or the provocation of failures during the loading of the implant at the delivery system.

The replacement valve may comprise between two and five leaflets. Such valves have been found to be beneficial when placed at the aortic valve.

A respective anti-leakage skirt may surround the first and second ends. Such anti-leakage skirts, on the one hand facilitate the function of the respective part of the implant and also allow an improved attachment of the implant at the respective vessel to which it is intended to be attached.

The first end may have an outer diameter that is smaller than an outer diameter of the second end in the expanded state. Such diameters have been found to be required for accurate placement at the aortic valve and the aortic root.

A diameter of the implant may decrease between the first end and the central portion and a diameter of the implant may also decrease between the second end and the central portion in the expanded state. By forming the implant in this way an attachment of the implant at the aortic root can be improved.

An outer diameter of the main body may reduce in size between the first end up to a first point of transition and then increases in size toward the part of the central portion having the largest outer diameter, with the outer diameter of the main body decreasing in size from the part of the central portion having the largest outer diameter up to a second point of transition, and with the outer diameter of the main body increasing in size between the second point in transition and the second end. Such a shape is believed to be particularly suitable for a minimally invasive treatment of acute type A dissection, ascending aorta aneurysm, or other conditions that require urgent intervention.

The main body may comprise two stent frames with the first stent frame having the replacement valve attached therein and the second stent frame comprising the self-expandable aortic stent frame, the first stent frame extending from the first end to beyond the part of the central portion having the largest outer diameter.

The expansion of the implant may be made possible by the respective first and second stent frames used. The respective first and second stent frames may each include a plurality of arms that are interconnected in such a way that following the expansion of the implant into the expanded state they adapt to the anatomical need of the location of the implant. In this way the design of such a stent frame is adapted to accommodate the anatomical needs and implantation locations.

The first and second stent frames may e.g. comprise e.g. a balloon or self-expandable stent frame. Moreover, between 6 and 50 arms may be provided to form the respective stent frames.

Similarly the internal branches may comprise further stent frames which can be deployed in the coronary arteries adjoining the aortic valve. The stent frames of the internal branches may likewise be formed with a plurality of arms and from the same or similar materials as the first and second stent frames.

In this connection it should be noted that each of the first and second stent frames may be designed from a collapsible and flexible metal. The material can in this respect be superelastic, a shape memory alloy, self-expandable (e.g. Nitinol), bal-Ion expandable (e.g. Cobalt chromium, Stainless Steel, etc) or polymeric or artificial. In order to manufacture the first and second stent frames, these can be laser cut form a piece of tube, the first and second stent frames can also be braided.

Additionally or alternatively the different sections of the first and second stent frames can be made of different materials or continuous material, with the different materials then being selected in dependence on the desired purpose. For example, the fist stent frame for placement at the ascending aorta may be designed more flexible than the second stent frame as this is designed to hold and support the replacement valve at the aortic valve.

Additional anchoring of the implant to an artery can be accomplished by means of sutures, barbs or hooks that can be further provided at the respective first and second stent frame. Moreover, a radial force present in each section of the first and second stent frames can be adjusted via the materials selected for each section of the respective stent frame, the specific (outer) dimensions selected for each section of the respective stent frame or the processing of the specific sections may be dimensioned for placement at the location of the aortic valve and the ascending aorta.

The first stent frame like the second stent frame may comprises one or more eyelets for fixing the stent frame at the ascending aorta and the aortic valve respectively.

The first stent frame may extend from the first end to a height of the implant corresponding to 55% to 75%, in particular to 60% to 70%, especially to 62% to 68%, of a length of the implant between an opening at the first end of the implant and an opening at the second end of the implant.

The second stent frame may extend from an opening at the second end to the second transition. A length of the second stent frame may correspond to a length of 15 to 35%, in particular 20 to 30% and especially of 22 to 28% of a length of the implant between the opening at the second end and the opening at the first end.

A gap present between the first stent frame and the second stent frame may have a width that corresponds to 2 to 30%, in particular 5 to 20%, especially 6 to 15%, of a length of the implant between the opening at the second end and the opening at the first end.

Such lengths, sizes and dimensions of the respective sections are believed to be particularly suitable for a minimally invasive treatment of acute type A dissection, ascending aorta aneurysm, or other conditions that require urgent intervention.

The first exits from the internal branches may open into the central portion of the implant between the first point of transition and the part of the central portion having the largest outer diameter, in particular:
wherein openings of the first exits of the internal branches may have a diameter which corresponds to 30% to 70% of a length of the implant between the part of the central portion having the largest outer diameter and the first point of transition; and/or
wherein openings of the first exit from the internal branches may be arranged between 20% to 80% of a length of the implant between the part of the central portion having the largest outer diameter and the first point of transition.

Arranging the exits from the internal branches in this way is believed to be beneficial to conducting the minimally invasive surgery envisaged for this implant.

The first end may have an at least substantially cylindrical outer shape between an opening of the implant and the part of the main body reducing in size between the first end and the first point of transition, optionally wherein the first end may have an at least substantially cylindrical outer shape at an opening of the replacement valve, in particular wherein the replacement valve may be arranged within the at least substantially cylindrical outer shape. Such shapes are known to be beneficial as shapes of valves used to replace the aortic valve.

According to a further aspect the above object is also satisfied by delivery device for an implant, the delivery device comprising;
a flush port;
a main body part for holding, inflating and/or releasing the implant;
one or more guidewires for holding, inflating and/or releasing one or more internal branches of the implant; and
an actuation mechanism for moving the implant to a delivery site.

The delivery device may have an apparatus to pre-catheterize the internal branches that are used to connect the coronary arteries to the implant. Such an apparatus saves time during the procedure, avoiding manipulation of guidewires and catheters inside the implanted implant.

The delivery device may be preloaded with an implant so that this can be stored ready to use on a shelf in a medical facility to significantly reduce the treatment time of acute aortic syndromes leading to reduced mortality rates of acute aortic syndromes.

The implant and/or the delivery device may have one or more radiopaque markings to increase visibility under x-rays and to increase precision and accuracy during deployment.

The one or more guidewires can be retracted from the main body on pre-catheterization of the internal branches at the delivery site.

The actuation mechanism may have a torque control and may be able to rotate the implant about an axis of the main body. In this way the implant can be positioned in relation to the extremities in an as good as possible manner at the aortic valve, to deploy the valve and stent at the desired deliver site.

The delivery device may be improved with steerable control, to increase precision and accuracy at deployment.

The delivery device may have a knob or the like at the actuation mechanism, with the knob in particular being able to be rotated about an axis of rotation of the actuation mechanism. By turning the knob the lumen is able to deflect and allows a better positioning of the tip and the main body of the delivery device and less stress over the system during deployment. This leads to an improved accuracy of deployment of the delivery device and hence of an implant that is delivered to a delivery site using the delivery device.

According to a further aspect the above object is also satisfied by a method of delivering an implant using a delivery device, the method comprising the steps of:
- moving the implant to a delivery site by means of the delivery device;
- pre-catheterizing the internal branches of the implant at the delivery site;
- positioning the main body of the implant at the delivery site; and
- deploying the implant at the delivery site.

Such a minimally invasive procedure is believed to significantly reduce the complications known from the Bentall-de Bono procedure.

The invention will be described in detail by means of embodiments and with reference to the drawings. These show preferred deployment locations and embodiments. The features described may be configured in various combinations, which are encompassed in this document. The drawings show:
- Fig. 1: a side view of an implant;
- Fig. 2: a perspective view of a delivery device having an implant arranged thereat; and
- Fig. 3: a further perspective view of the delivery device of Fig. 2 showing positions a tip thereof can occupy when an actuation mechanism of the delivery device is activated.

In the following the same reference numerals will be used for parts having the same or equivalent function. Any statements made having regard to the direction of a component are made relative to the position shown in the drawing and can naturally vary in the actual position of application.

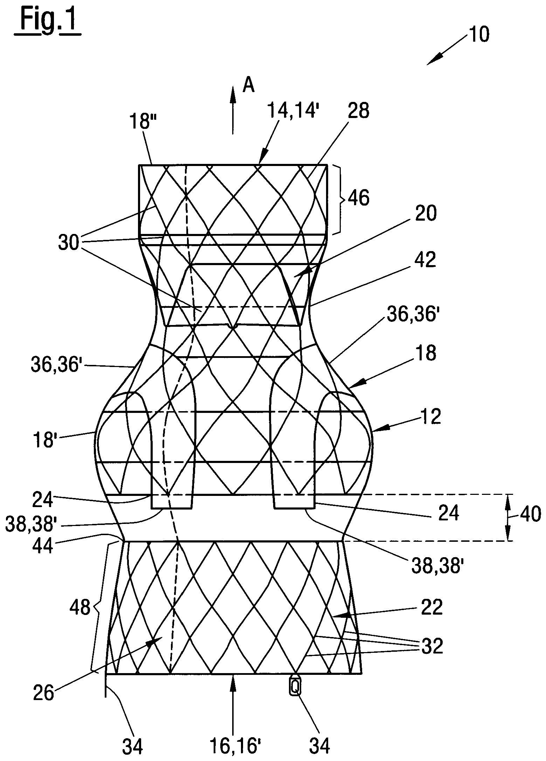

Fig. 1 shows an implant 10 for placement in a cardiac vessel, the implant 10 being able to be expand from an un-deployed state to an expanded state in which the implant 10 has a larger outer diameter than in the storage state. The implant 10 has a main body 12 with first and second ends 14, 16. A central portion 18 is arranged between the first and second ends 14, 16. The central portion 18 of the implant 10 has a larger outer diameter than the first and second ends 14, 16 of the implant 10 respectively when the implant 10 is in the expanded state.

The implant 10 further comprises a replacement valve 20 for placement at the aortic valve arranged within the first end 14 of the implant 10. A passage 18" extends from the first end 14 to the second end 16, with the replacement valve 20 being arranged within the passage 18".

A self-expandable aortic stent frame 22 for placement at the ascending aorta is arranged within the second end 16 of the implant 10. Furthermore, two internal branches 24 extend from the main body 12. The internal branches 24 are configured to be attached to either the coronary arteries, or if provided a respective stent arranged within the coronary arteries (both not shown).

The implant is completely covered with a material 26. The material 26 covers two stent frames 22, 28 with the first stent frame 28 having the replacement valve 20 attached therein and the second stent frame 22 comprising the self-expandable aortic stent frame.

The first stent frame 28 extends from an opening 14' of the first end 14 to beyond a part 18' of the central portion 18 having the largest outer diameter. A gap 40 is present between the first stent frame 28 and the second stent frame 22, with the material 26 bridging the gap 40 between the first and second stent frames 28, 22.

Each of the first and second stent frames 22, 28 comprises a plurality of respective arms 30, 32 which are indicated in a shaded manner in Fig. 1. The arms are interconnected to form the respective expandable stent frame 22, 28. The second stent frame 22 extends from an opening 16' at the second end 16 to the second transition 44.

Eyelets 34 are visible at the second end. The eyelets 34 are provided at the second stent frame 22 in order to facilitate the attachment of the implant 10 to the aortic root.

The valve 20 may be a dry tissue valve and may, for example, comprise between two and five leaflets. In order to prevent leaks from arising between the valve 20 and the part of the aorta at which it is placed, the implant 10 comprises an anti-leakage skirt 46 that is arranged to cover that part of the first end 14 in which the valve 20 is arranged.

Similarly an anti-leakage skirt 48 may be arranged to surround that part of the second end 16 which is configured to contact the aortic root.

The anti-leakage skirt 48, like the anti-leakage skirt 46 may be formed from one and the same material 26, the material 26 may then extend on an outer surface of the respective first and second stent frames 28, 22 between the first end 14 and the second end 16.

The material 26 may also cover the internal branches 24, so that the complete implant 10 is covered with one piece of material 26 that then preferably does not comprise any holes, leaks or the like.

The first end 14 has an outer diameter that is smaller than an outer diameter of the second end 16 in the expanded state, with an outer diameter of the main body 12 reducing in size between the first end 14 up to a first point of transition 42 and then increases in size toward the part 18' of the central portion 18 having the largest outer diameter, with the outer diameter of the main body 12 decreasing in size from the part 18' of the central portion 18 having the largest outer diameter up to a second point of transition 44. The outer diameter of the main body 12 then increases in size between the second point 44 in transition and the second end 16.

The first end 14 of the implant 10 can be defined as that part of the implant that extends between the opening 14' and the first point of transition 42.

The central portion of the implant 10 can be defined as that part of the implant 10 that extends between the first point of transition 42 and the second point of transition 44.

The second end 16 of the implant 10 can be defined as that part of the implant 10 that extends between the second point of transition 44 and the opening 14'.

The internal branches 24 each have a first exit 36 with an opening 36' and a second exit 38 with an opening 38'. The openings 36' each open into the central portion 18 of the implant 10. The openings 38' present at the second exits 38 each open into an area surrounding the implant 10.

The first exits from the internal branches 24 open into the central portion 18 of the implant 10 between the first point of transition 42 and the part of the central portion 18 having the largest outer diameter.

The first end 14 has an at least substantially cylindrical outer shape between the opening 14' of the implant 10 and the part of the main body 12 reducing in size between the first end 14 and the first point of transition 42.

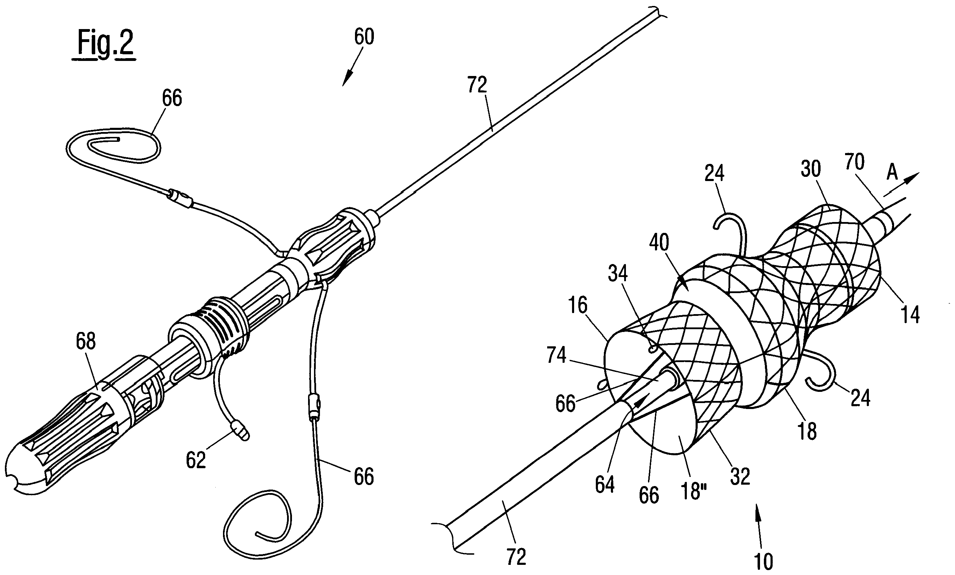

Fig. 2 shows a delivery device 60 for the implant 10 also known as a tri-axial catheter. The delivery device 60 comprises a flush port 62 for flushing internal cavities of the delivery device (not shown), a main body 64 for holding, inflating and/or releasing the implant 10, one or more guidewires 66 for holding, inflating and/or releasing one or more internal branches 24 of the implant 10, and an actuation mechanism 68 for moving the implant 10 to a delivery site.

The main body 64 is arranged at an inner shaft 74 at an end of the lumen 70 that extends between the actuation mechanism 68 and a tip 72 of the tri-axial catheter 60. On delivery of the implant 10 at the delivery site, the internal branches are pre-catheterized, e.g. using a balloon catheter and are thereby connected to the coronary arteries and/or stent frames present in the coronary arteries. Following the attachment of the internal branches 24 at the coronary arteries the guidewires 66 can be retracted from the main body 64.

The implant 10 can be guided to the delivery site by means of the actuation mechanism 68 that has a torque control and can rotate the implant 10 about an axis A of the main body 12. The axis A extends between the openings 14', 16' of the main body 12 of the implant 10.

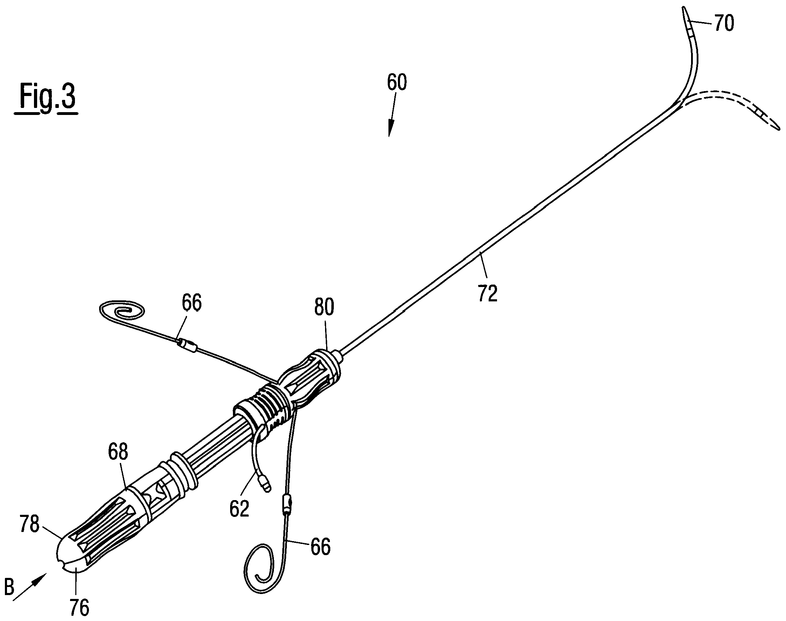

Fig. 3 shows a perspective view of the delivery device 60 of Fig. 2. The actuation mechanism 68 comprises a knob 76 at a distal end 78 of the actuation mechanism 68. On turning the knob 68 about an axis B of the actuation mechanism 68 which extends between the distal end 78 and a proximal end 80 of the actuation mechanism 68, the lumen 72 is able to deflect. This enables the tip 70 of the lumen 72 to occupy various positions, of which two are indicated in Fig. 3. Such a deflection of the lumen 72 reduces the overall stress in the system which leads to a better positioning of the implant 10 at a delivery site and an overall improved accuracy of deployment of the delivery device 60 and hence of an implant 10 that is delivered to a delivery site using the delivery device 60.

On delivering the implant 10 to the delivery site, i.e. the heart, by means of the delivery device 60, the arterial femoral access is preferably used. The lumen 72 is then moved through the arteries to the heart.

The position of the implant 10 and/or of the delivery device 60 may be tracked using e.g. x-rays in order to monitor radiopaque markings present at the implant 10 and/or the delivery device 60. Thereby the precision and accuracy during deployment of the implant 10 within the heart can be further increased, so that the replacement valve 20 can be positioned accurately at the aortic valve.

### List of reference numerals:

- 10: implant
- 12: main body
- 14, 14': first end, opening at 14
- 16, 16': second end, opening at 16
- 18, 18', 18": central portion, part of 18 having largest outer diameter of 10, passage
- 20: valve
- 22: second stent frame
- 24: branches
- 26: material
- 28: first stent frame
- 30: arms
- 32: arms
- 34: eyelets
- 36, 36': first exit, opening of 36
- 38, 38': second exit, opening of 38
- 40: gap
- 42: first point of transition
- 44: second point of transition
- 46: anti-leakage skirt
- 48: anti-leakage skirt
- 60: delivery device
- 62: flush port
- 64: main body part of 60
- 66: guidewire
- 68: actuation mechanism
- 70: tip
- 72: lumen
- 74: inner shaft
- 76: knob
- 78: distal end

- A: axis of 10
- B: axis of 68

## Claims

1. An implant (10) for placement in a cardiac vessel, the implant (10) being able to be expanded from an un-deployed state to an expanded state in which the implant (10) has a larger outer diameter than in the storage state, the implant (10) having a main body (12) with first and second ends (14, 16) and a central portion (18) arranged between the first and second ends (14, 16), the central portion (18) of the implant (10) having a larger outer diameter than the first and second ends (14, 16) of the implant (10) when the implant (10) is in the expanded state, the implant (10) further comprising:
- a replacement valve (20) arranged at the first end (14) of the implant (10) for placement at the aortic valve,
- a self-expandable aortic stent frame (22) arranged at the second end (16) of the implant (10) for placement at the ascending aorta; and
- two or more internal branches (24) for connection to further vessels, the internal branches (24) extending from the main body (12).

2. An implant (10) according to claim 1,
wherein the main body (12) is completely covered with a material (26), optionally wherein the internal branches (24) and in particular the complete implant (10) is covered with material (26).

3. An implant (10) according to claim 1 or claim 2,
wherein respective first exits (36) of the internal branches (24) have an opening (36') that opens into the central portion (18) of the implant (10) and respective second exits (38) of the internal branches (24) have an opening (38') that opens into an area surrounding the implant (10).

4. An implant (10) according to one of the preceding claims,
wherein the valve (20) is a dry tissue valve; and/or
wherein a respective anti-leakage skirt (46, 48) surrounds the first and second ends (14, 16); and/or
wherein the replacement valve (20) comprises between two and five leaflets.

5. An implant (10) according to one of the preceding claims,
wherein the first end (14) has an outer diameter that is smaller than an outer diameter of the second end (16) in the expanded state; and/or
wherein a diameter of the implant (10) decreases between the first end (14) and the central portion (18) and between the second end (16) and the central portion (18) in the expanded state.

6. An implant (10) according to one of the preceding claims,
wherein an outer diameter of the main body (12) reduces in size between the first end (14) up to a first point of transition (42) and then increases in size toward the part (18') of the central portion (18) having the largest outer diameter, with the outer diameter of the main body (12) decreasing in size from the part of the central portion (18) having the largest outer diameter up to a second point of transition (44), and with the outer diameter of the main body (12) increasing in size between the second point (44) in transition and the second end (16).

7. An implant (10) according to one of the preceding claims,
wherein the main body (12) comprises two stent frames (22, 28) with the first stent frame (28) having the replacement valve (20) attached therein and the second stent frame (22) comprising the self-expandable aortic stent frame, the first stent frame (28) extending from the first end (14) to beyond the part (18') of the central portion (18) having the largest outer diameter.

8. An implant (10) according to claim 7,
wherein the first stent frame (28) extends from the first end (14) to a height of the implant (10) corresponding to 55% to 75%, in particular to 60% to 70%, especially to 62% to 68%, of a length of the implant (10) between an opening (14') at the first end (14) of the implant (10) and an opening (16') at the second end (16) of the implant (10).

9. An implant (10) according to claim 7 or claim 8,
wherein the second stent frame (22) extends from an opening (16') at the second end (16) to the second transition (44); and/or
wherein a length of the second stent frame (22) corresponds to a length of 15 to 35%, in particular 20 to 30% and especially of 22 to 28% of a length of the implant (10) between the opening (16') at the second end (16) and the opening (14') at the first end (14); and/or
wherein a gap (40) present between the first stent frame (28) and the second stent frame (22) has a width that corresponds to 2 to 30%, in particular 5 to 20%, especially 6 to 15%, of a length of the implant (10) between the opening (16') at the second end (16) and the opening (14') at the first end (14), optionally wherein the gap (40) between the first and second stent frames (28, 22) is bridged with material (26).

10. An implant (10) according to one of the preceding claims 7 to 9 and 3, wherein the first exits from the internal branches (24) open into the central portion (18) of the implant (10) between the first point of transition and the part of the central portion (18) having the largest outer diameter, in particular:
wherein openings of the first exits of the internal branches (24) have a diameter which corresponds to 30% to 70% of a length of the implant (10) between the part (18') of the central portion (18) having the largest outer diameter and the first point of transition (42); and/or
wherein openings (36') of the first exit (36) from the internal branches (24) are arranged between 20% to 80% of a length of the implant (10) between the part (18') of the central portion (18) having the largest outer diameter and the first point of transition.

11. An implant (10) according to one of the preceding claims 6 to 10,
wherein the first end (14) has an at least substantially cylindrical outer shape between an opening (14') of the implant (10) and the part of the main body (12) reducing in size between the first end (14) and the first point of transition (42), optionally wherein the first end (14) has an at least substantially cylindrical outer shape at an opening of the replacement valve (20), in particular wherein the replacement valve (20) is arranged within the at least substantially cylindrical outer shape.

12. A delivery device (60) for an implant (10), in particular an implant (10) in accordance with at least one of the preceding claims, the delivery device (60) comprising;
a flush port (62);
a main body (64) part for holding, inflating and/or releasing the implant (10); one or more guidewires (66) for holding, inflating and/or releasing one or more internal branches (24) of the implant (10); and
an actuation mechanism (68) for moving the implant (10) to a delivery site.

13. A delivery device according to claim 12,
wherein the one or more guidewires (66) can be retracted from the main body (64) on pre-catheterization of the internal branches (24) at the delivery site.

14. A delivery device according to claim 12 or claim 13,
wherein the actuation mechanism (68) has a torque control and can rotate the implant (10) about an axis of the main body (12); and/or wherein the actuation mechanism (68) comprises a knob (76) that can be rotated about an axis of rotation (B) of the actuation mechanism (68).

15. A method of delivering an implant (10) in accordance with one of claims 1 to 9 using a delivery device (60) according to one of claims 10 to 14, the method comprising the steps of:
- moving the implant (10) to a delivery site by means of the delivery device (60);
- pre-catheterizing the internal branches (24) of the implant (10) at the delivery site;
- positioning the main body (12) of the implant (10) at the delivery site; and
- deploying the implant (10) at the delivery site.
